# EUROPÄISCHE PATENTSCHRIFT

(11) **EP 0 912 177 B1**
(45) Veröffentlichungstag und Bekanntmachung des Hinweises auf die Patenterteilung: **21.01.2004**
(21) Anmeldenummer: 97929212.5
(22) Anmeldetag: 18.06.1997
(51) Int. Cl.: A61K 31/44, A61P 13/12, A61P 9/10, A61P 11/00

(54) **VERWENDUNG VON FLUPIRTIN ZUR THERAPIE UND PROPHYLAXE VON MYOKARDINFARKT, SCHOCKNIERE UND SCHOCKLUNGE**
USE OF FLUPIRTINE FOR THERAPY AND PROPHYLAXIS OF MYOCARDIAL INFARCTION, SHOCK KIDNEY AND SHOCK LUNG
UTILISATION DE FLUPIRTINE POUR LA THERAPIE ET PREVENTION DE L'INFARCTUS DU MYOCARDE, POUMON DE CHOC ET REIN DE CHOC

(30) Priorität: 27.06.1996 DE 19625582
(43) Veröffentlichungstag der Anmeldung: 06.05.1999
(73) Patentinhaber: VIATRIS GmbH & Co. KG, 60314 Frankfurt am Main (DE)
(72) Erfinder: PERGANDE, Gabriele, D-63071 Offenbach (DE); MÜLLER, Werner, E., D-65203 Wiesbaden (DE); OSBORNE, Neville, Oxford OX8 1JS (GB); ULRICH, Heinz, D-63843 Niedernberg (DE)
(86) Internationale Anmeldenummer: PCT/EP1997/003179
(87) Internationale Veröffentlichungsnummer: WO 1997/049398

(56) Entgegenhaltungen:
- WO-A-97/17072
- DE-A- 4 327 516
- SHANDRA ET AL.: "Experimental brain trauma: effects of vitamin treatment" J. NEUROTRAUMA, Bd. 12, Nr. 3, 1995, Seite 489 XP002043184
- OSBORNE ET AL.: "Protection of rabbit retina from ischemic injury by flupirtine" INVEST. OPHTHALM. VIS. SCI., Bd. 37, Nr. 2, Februar 1996, Seiten 274-280, XP002043185
- B. GROOS: "Analgetikum Flupirtin schützt vor dem Zelltod" T W NEUROLOGIE PSYCHIATRIE, Bd. 9, Nr. 10, 1995, Seite 606 XP000673251
- S PEROVIC ET AL.: "Flupirtine increases the levels of glutathione and Bc1-2 in hNT (human Ntera/D1) neurons: mode of action of the drug-mediated anti-apoptotic effect" EUR. J. PHARMACOL., Bd. 317, Nr. 1, 12.Dezember 1996, Seiten 157-164, XP000673257
- POWELL-JACKSON ET AL.: "Use of flupirtine maleate as an analgesic in patients with liver disease" BR. J. CLIN. PRACT., Bd. 39, Nr. 2, 1985, Seiten 63-66, XP002043186

## Beschreibung

Die Erfindung betrifft die Verwendung von Flupirtin oder dessen Salze als Arzneimittel zur Prophylaxe und Therapie von Myokardinfarkt, Schockniere und Schocklunge.

Flupirtin ist ein bekanntes eingeführtes zentral wirksames, nicht-opiates Analgetikum, das in Deutschland u.a. für die Therapie von Nervenschmerzen, Schmerzen bei abnutzungsbedingten Gelenkerkrankungen, Kopfschmerzen und postoperativen Schmerzen zugelassen ist.

Seine analgetischen Wirkungen entfaltet Flupirtin über andere Wirkmechanismen als die Opiat/Opioid-Analgetika (Nickel, B., Postgrad, Med. J. 63 (Suppl.3), 19 (1987) ; Szelenyi, I., Nickel, B., Borbe, H. O., Brune K., Br. J. Pharmacol. 143,89 (1989)). In elektrophysiologischen Untersuchungen wurde gezeigt, daß Flupirtin sowohl auf der supraspinalen als auch auf der spinalen Ebene in das nozizeptive Geschehen einzugreifen vermag (Carisson, K. H., Jurna, I., Eur. J. Pharmakol. 143,89 (1987); Bleyer, H., Carlsson K. H., Erkel, H. J.,Jurna, I., Eur. J. Pharmacol. 151,259 (1988); Nickel, B., Aledter, A., Postgrad Med. J. 63 (Suppl.3) 41 (1987)).

Neben guten analgetische Eigenschaften verfügt Flupirtin über muskelrelaxierende Eigenschaften, so daß Flupirtin auch für die Behandlung von Muskelverspannungen oder bei Erkrankungen, die auf Muskelverspannungen beruhen, eingesetzt werden kann(DE 40 22 442).

Desweiteren wurde bei Untersuchungen der muskelrelaxierenden Wirkung von Flupirtin an der Ratte gefunden, daß die Flupirtin-Wirkung sich durch die exitatorische Aminosäure N-Methyl-D-Aspartat (NMDA) hemmen läßt. Aufgrund dieser NMDA-antagonistischen Wirkung ist Flupirtin auch zur Behandlung von NMDA-vermittelten ZNS-Erkrankungen, wie zum Beispiel zerebraler Ischämie, neurodegenerativer Erkrankungen, epileptischer Anfälle geeignet(DE 43 27 516).

Chemisch gesehen handelt es sich bei Flupirtin um 2-Amino-3-ethoxycarbonylamino-6-(p-fluor-benzylamino)-pyridin.

Die Synthese von Flupirtin und dessen pharmazeutisch verwendbaren Salze wird in den Patenten DE 17 95 858, DE 31 33 519 und DE 34 16 609 beschrieben.

Überraschenderweise wurde nun gefunden, daß Flupirtin in der Lage ist, die Expression von Bcl-2 zu erhöhen sowie nekrotische Prozesse zu hemmen.

Damit eröffnet sich die Möglichkeit, Flupirtin zur Behandlung von Erkrankungen, die mit einer unphysiologisch hohen Zellsterberate einhergehen, einzusetzen.

Insbesondere können damit Erkrankungen , die mit lokalem Gewebstod als schwerste Folge einer örtlichen Stoffwechselstörung beispielsweise infolge von hypoxischer, mechanischer, thermischer, toxischer oder Strahlenbelastung verbunden sind, behandelt werden.

Im Organismus kann das Absterben von Zellen auf zwei verschiedenen wegen erfolgen: durch Apoptose oder Nekrose.

Während die Apoptose (programmierter Zelltod, "Zell-Suizid") einen aktiven Prozeß der Zellzerstörung darstellt, ist die Nekrose Folge einer unspezifischen Schädigung insbesondere der Zellmembran.

Die pathologischen Charakteristika der Nekrose differieren von denen der Apoptose. Die Apoptose ist gekennzeichnet durch eine Schrumpfung der Zelle, Kondensation des Chromatins sowie charakteristische Ausstülpungen der Zellmembran. Auf DNA-Ebene erfolgt durch Endonukleasen eine Fragmentierung in 180 Basenpaar große Bruchstücke (charakteristisches Leitermuster in der Gelelektrophorese). Die Zelle zerfällt, ohne Freisetzung von Bestandteilen des Zytoplasmas, in membranumschlossene Apoptosekörper. Diese werden durch ihre modifizierte Oberflächenstruktur von spezifischen Rezeptoren ortsständiger Makrophagen bzw. Epithelzellen schnell erkannt und komplett phagozytiert, eine Entzündungsreaktion bleibt typischerweise aus (Amling et al. 1994, Savill, 1994, Kerr et al. 1994).

Im Gegensatz zur Apoptose wird beim Zelltod durch Nekrose die Plasmamembran zerstört und der Zellinhalt in den Extrazellularraum freigesetzt. Dies führt zu Gewebeschädigungen und Entzündungsreaktionen (Savill, 1994, Kerr et al. 1994). Die Zellzerstörung ist mit der Freisetzung von Lactatdehydrogenase (LDH) verbunden. Die LDH ist ein zytoplasmatisches Enzym und Bestandteil aller Gewebe. Bei Organschäden kann sie vermehrt ins Plasma übertreten und ist bei vielen pathologischen Zuständen, u.a. nach Myokardinfarkt, bei akuter Hepatitis oder toxischen Leberschäden erhöht.

Nicht nur die Art und die Intensität/Dauer des Zelltodbewirkenden Prozesses ist entscheidend, ob es zu einer apoptotischen oder nekrotischen Schädigung kommt; nekrotische und apoptotische Prozesse können im Gewebe auch gleichzeitig auftreten (Yoshimura et al. 1996).

Aus der Literatur ist bekannt, daß der programmierte Zelltod (Apoptose) durch Bcl-2, ein 25 kDa membranständiges Protein mit 239 Aminosäuren gehemmt werden kann. Das Protein ist in der Kernmembran, Teilen des endoplasmatischen Retikulums und der äußeren und inneren Mitochondrienmembran lokalisiert. Der Wirkmechanismus von Bcl-2 ist noch nicht vollständig geklärt, u.a. werden folgende mögliche Mechanismen diskutiert: Modulation der mitochondrialen Funktion, indirekte antioxidative Wirkung, direkte Verhinderung der Chromatin-Spaltung, Regulation der cytosolischen Ca²⁺ Konzentration, Modulation des Transports von Proteinen durch die Kern-Membran, indirekt antiapoptotische Wirkung durch Hemmung des Apoptose-induzierenden Proteins Bax (Hale et al. 1996, Park et al. 1996).

Die DE-A-4327516 offenbart die Verwendung von Flupirtin bei u.a. amyotropher Lateralsklerose und Bronchospasmen. Aus J. Neurotrauma, 1995, 12(3): 489 und Invest. Ophthalm. Vis. Sci., Feb. 1996, 37(2): 274-280 ist die Verwendung von Flupirtin bei verschiedenen Krankheiten wie u.a. cerebrale Ischämie, toxische Zellschäden nach neurodegenerativen Erkrankungen, Gehirntrauma und retinaler Ischämie bekannt. Das Dokument T.W. Neurologie Psychiatrie, 1995, 9(10): 606 weist zusätzlich auf einen vorteilhaften Einfluß von Flupirtin bei Zellschäden durch Apoptose hin.

Gegenstand der vorliegenden Erfindung ist insbesondere gegenüber dem vorstehenden genannten Stand der Technik die Verwendung des Wirkstoffes Flupirtin oder dessen pharmazeutisch verwendbaren Salzes zur Herstellung eines Arzneimittels zur Therapie und Prophylaxe von Myokardinfarkt, Schockniere und Schocklunge.

Anhand der pharmakologischen Untersuchungen soll die neue erfindungsgemäße Wirkung von Flupirtin näher erläutert werden.

### Pharmakologische Untersuchungen

### Einfluß von Flupirtin auf die BCL-2 Expression

### Material und Methoden

### Material

Folgende Reagenzien wurden bezogen: Poly-L-Lysin (Mᵣ > 300, 000) , Glycin, (3-[4,5-Dimethylthiazol-2-yl]-2,5-Diphenyltetrazolium Bromid; Thiazolyl Blau) [MTT], Dulbecco's Modified Eagle's Medium (DMEM/HG; enthaltend 4.5 g/l Glukose ohne Glutamin) und Minimum Essential Medium-Eagle [ohne Methionin, Cystein und L-Glutamin] (MEM) von Sigma (St. Louis, MO, USA); L-Glutamin von Biochrom (Berlin); [³⁵S]Methionin/Cystein von ICN-Radiochemicals (Irvine, CA; USA) und monoklonale Antikörper gegen menschliches Bcl-2 (gezogen in der Maus) von Santa Cruz Biotechnology (Santa Cruz, CA, USA).

Flupirtinmaleat [2-Amino-3-Ethoxycarbonylamino-6-(4-Fluor-Benzylamino)-Pyridin Maleat] (Mᵣ: 420,41) (ASTA-Medica AG). hNT Neuronen, hNT Neuronen konditioniertes Medium und hNT Neuronen Inhibitions Medium (Stratagene, Heidelberg).

### hNT Neurone

hNT Neurone wurden wie beschrieben (Younkin et al., 1993; Pleasure und Lee, 1993) gezüchtet. Sie wurden in der ersten Zeit in hNT Neuronen-Inhibitions-Medium und während der drei folgenden Wochen in hNT Neuronen-konditioniertem-Medium, wie in der Gebrauchsanweisung der Fa. Stratagene beschrieben, kultiviert.
hNT Neurone [vier Wochen alt] (Younkin et al., 1993) wurden mit verschiedenen Konzentrationen von Glutamat in Salzlösung, die 120 mM NaCl, 5,4 mM KCl, 1,8 mM CaCl₂, 15 mM Glucose and 25 mM Hepes [pH 7,4] enthielt, behandelt. In den Experimenten wurde die Salzlösung zusätzlich mit 50 µM Glycin supplementiert.
Die Zellen wurden mit Glutamat für 30 min bei 37° C inkubiert. Nach der Inkubation wurde die Salzlösung entfernt und mit DMEM/HG, das 2 mM Glutamin, 100 mU/l Insulin, 0,1 U/ml Penicillin and 0,1 U/ml Streptomycin enthielt, ersetzt. Die Inkubation wurde für weitere 24 Stunden fortgesetzt.
Flupirtin wurde den Zellen 2 Stunden vor Hinzufügen von Glutamat gegeben.

### Metabolische Markierung und quantitative Bestimmung der Immunpräzipitation von Bcl-2

hNT Neurone wurden für 6 Stunden in MEM [ohne Methionin, Cystein und L-Glutamin] ergänzt mit 1% bovinem Serum Albumin in Gegenwart von 100 µCi/ml [³⁵S]Methionin/Cystein metabolisch markiert. Die Zellen wurden geerntet und in Gegenwart von 0,4 % SDS und Trichloressigsäure lysiert. Antikörper-bindendes Material wurde mit dem monoklonalen anti-Bcl-2 Antikörper wie beschrieben (Oltvai et al., 1993; Dole et al., 1994) immunpräzipitiert. Das entstandene Immunpräzipitat wurde nach der Größe seiner Proteine mittels eines 12,5% SDS-Polyacrylamid Gels getrennt und wie beschrieben (Castle et al., 1993) weiter verarbeitet. Zur semiquantitativen Analyse der Banden auf dem Autoradiogramm, wurden die Banden mit einem integrierenden Densitometer (Shimadzu CS-910/C-R1A) abgetastet.

### Immunfärbung

Die Zellen wurden mit Methanol, das 0,02% Äthylen-Glycolbis(β-Aminoethyl-Äther) *N,N,N',N*'-Tetraessigsäure [EGTA] enthielt bei -20°C, wie beschrieben (Bachmann et al., 1986), fixiert. Anschließend wurden die Zellen mit monoklonalen Bcl-2 [gezogen, gegen menschliches Antigen] Antikörpern inkubiert; die Immunkomplexe wurden mit FITC markiertem anti-Maus Ig sichtbar gemacht.

### Ergebnisse

### Einfluß von Flupirtin auf die Expression von Bcl-2

Die Expression von Bcl-2 in hNT Neuronen wurde mit zwei verschiedenen Techniken bestimmt. Einmal wurde Bcl-2. mit der Mikrofluoreszens-Methode lokalisiert. Zellen, die entweder unbehandelt waren oder mit 10 µM Flupirtin für 2 Stunden vorbehandelt waren, wurden fixiert und mit Antikörpern gegen Bcl-2 inkubiert. Anschließend wurden die Immunkomplexe mit einem Fluoreszens-Mikroskop sichtbar gemacht.
Zellen, die mit Flupirtin behandelt wurden, zeigen ein deutlich intensiviertes Färbemuster für Bcl-2 gegenüber der Kontrollgruppe. Diese Beobachtung deutet darauf hin, daß Flupirtin die Bcl-2 Expression induziert.

Um diese Schlußfolgerung weiter zu untermauern wurde ein weiteres Verfahren, Immunpräzipitation von metabolisch markiertem Bcl-2, angewandt. hNT Neurone wurden mit [³⁵S]Methionin/Cystein inkubiert. Die entstandenen Immunkomplexe wurden mit monoklonalen anti-Bcl-2 Antikörpern präzipitiert und die Proteine nach ihrer Größe aufgetrennt. Im Autoradiogramm wird das 26 kDa Bcl-2 Protein in Extrakten aus Kontroll-Proben zusammen mit der 31 kDa Bande, die schon früher von Oltvai et al. (1993) beobachtet wurde, sichtbar. Setzt man die Intensität der 26 kDa Bcl-2 Bande gleich 100%, zeigen Zellen, die mit 1mM Glutamat inkubiert waren eine erheblich verminderte Expression von Bcl-2, ≈ 80%.
Inkubation von hNT Neuronen mit Glutamat zusammen mit steigenden Konzentrationen von Flupirtin (3 und 10 µM) führte zu einem > 6-fachen Anstieg von Bcl-2.

### Beeinflussung der Nekrose durch Flupirtin

### Material und Methoden:

### Corticale Nervenzellkultur:

Es wurde das Vorderhirn 16 - 18 Tage alter fetaler Ratten verwendet. Die Hirnhäute wurden entfernt und die dissoziierten corticalen Zellen (1,5 - 2 · 10⁶) auf 35 mm Petri-Schalen ausgesät, die mit 0,1 mg/ml Poly-D-Lysin beschichtet waren und anschließend mit serumhaltigem Medium (Dulbecco's Modified Eagle's Medium - DMEM -, versetzt mit 4 mM L-Glutamin, 100 U/ml Penicillin / 100 mg/ml Streptomycin, 10 % fetalem Kälberserum) geblockt wurden. Die Primärkulturen wurden in DMEM kultiviert, das mit L-Glutamin (4 mM) - Glucose (6 g/l), Penicillin (100 U/ml), Streptomycin (100 mg/ml) und 10 % "Hormon-Medium", dem Transferrin (1mg/ml), Insulin (250 mg/ml), Putrescin (6 10⁻⁴M), Natriumselenit ( 3 x 10⁻⁷M), Progesteron (2 · 10⁻⁷M) und Estradiol (10⁻¹¹M) zugesetzt wurden.
Die Kulturen wurden mindestens 7 Tage in diesem Medium bei 5 % CO₂ / 95 % Luft in feuchtigkeitsgesättigter Atmosphäre bei 37°C gehalten.

### Hypoxie und Reoxygenierung:

7 Tage nach dem Aussäen wurden die corticalen Neurone in DMEM 1 g/ml; enthaltend: Glucose mit L-Glutamin und Penicillin / Streptomycin ohne "Hormon-Medium" in anoxischer Atmosphäre (95 % N / 5 % CO₂) bei 37°C für 5 h inkubiert. Zur Reoxygenierung wurden die Zellen für 3 h normoxischen Bedingungen (95 % Luft / 5 % CO₂) ausgesetzt. Die Kontrollkulturen wurden unter normoxischen Bedingungen gehalten. Flupirtin wurde der Kultur vor der Hypoxie zugesetzt.

### Lactatdehydrogenase-Assay:

Die Zellschädigung wurde über die Lactatdehydrogenase-(LDH) Freisetzung im Zellkultur-überstand nach Hypoxie / Reoxygenierung, gemessen.
Die LDH-Aktivität wurde spektrophotometrisch bestimmt; Pyruvat (0,6 mM) wurde bei 25°C in Phosphatpuffer pH 7,5 mit reduziertem Nicotinamid-Adenosin-Dinucleotid (NADH) (0,18 mM) in Anwesenheit der zu untersuchenden Probe (50 mM in 1,5 ml) inkubiert und der NADH-Verbrauch über eine Zeitspanne von 2 min. bei 340 nm bestimmt.

### Ergebnisse:

Nekrotische Vorgänge sind mit einer erhöhten Freisetzung von Laktat-Dehydrogenase [LDH] verbunden. Wie Tabelle 1 zu entnehmen ist, erfolgt nach Aussetzen der Zellen in eine hypoxische Atmosphäre und nachfolgende Reoxygenierung eine Erhöhung der LDH-Freisetzung um mehr als 50 %. Durch Vorbehandlung mit Flupirtin wird die durch Hypoxie und nachfolgende Reoxygenierung induzierte Nekrose verhindert wie aus der fehlenden Erhöhung der LDH-Freisetzung gegenüber der Kontrolle zu entnehmen ist (p < 0.05).

**Tabelle 1**

| Erhöhung der LDH-Freisetzung (Kontrollwert: 21,13 ± 3,14 mU/ml/mg Protein) | |
|---|---|
| Behandlung | Zunahme der LDH-Freisetzung im Vergleich zur Kontrolle (mU/ml/mg Protein) |
| Hypoxie 5 h + Reoxygenierung 3 h | 13,52 ± 2,85 |
| Hypoxie 5 h + Reoxygenierung 3 h + Flupirtin 100 µM | 1,19 ± 1,03 * |
| Einseitiger ANOVA-Test, gefolgt vom Dunnet-Test | |

| | |
|---|---|
| * p < 0,05 | |

Diese Ergebnisse zeigen deutlich, daß Flupirtin ein vielversprechendes Arzneimittel insbesondere zur Behandlung von Organerkrankungen mit zellzerstörenden Prozessen, wie z. B. Myokardinfarkt, Schockniere, Schocklunge, senile Makuladegeneration oder Traumata infolge mechanischer, thermischer, Strahlen- oder toxischer Einflüsse geeignet ist.

In mehreren in vitro- und in vivo-Modellen konnte für Flupirtin eine NMDA-antagonistische und damit verbundene neuroprotektive Wirkung nachgewiesen werden. Ein klinischer Einsatz von Flupirtin ist aufgrund dieser Daten u.a. denkbar bei der AIDS-Enzephalopathie. Für die Entstehung der AIDS-Enzephalopathie wird diskutiert, daß infizierte Makrophagen u.a. NMDA-agonistisch wirkende Neurotoxine freisetzen, die dann über eine Stimulation des NMDA-Rezeptors zum Nervenzelltod führen (Lipton 1992).

In Zellkulturversuchen konnte nachgewiesen werden, daß sowohl die NMDA- als auch die HIV (gp120)-vermittelte neurotoxische Wirkung durch Flupirtin gehemmt werden kann (Perovic et al. 1994).

Es war völlig überraschend, daß Flupirtin in der Lage ist, die Expression des Apoptose-hemmenden Proteins Bcl-2, dessen Vorkommen nicht nur auf Nervenzellen begrenzt ist, zu erhöhen. Bcl-2 ist in der Kernmembran, Teilen des endoplasmatischen Retikulums und der Mitochondrienmembran lokalisiert.

Darüber hinaus wurde ebenso überraschend gefunden, daß Flupirtin in vitro auch den nekrotischen Zelltod zu hemmen vermag, wie die reduzierte Freisetzung des cytoplasmatischen Enzyms Lactatdehydrogenase belegt. Erhöhungen der Lactactdehydrogenase treten beim Menschen bekanntermaßen infolge von zellzerstörenden Prozessen u.a. nach Myokardinfarkt, bei akuter Hepatitis oder toxischen Leberschäden auf.

Flupirtin kann zur Prophylaxe und Therapie in bekannter Weise in folgenden Darreichungsformen verabreicht werden:

Tabletten, Filmtabletten, Hartgelatinekapseln, Weichgelatinekapseln, Pellets, Granulate, Dragees, Supositorien, Mikrokapseln, wäßrige oder ölige Suspensionen, ölige Lösungen, Injektionslösungen zur intramuskulären Verabreichung, Injektionslösungen zur intravenösen Verabreichung.

Geeignete Salze für die Herstellung des Arzneimittels sind alle physiologisch verträglichen Salze des Flupirtins. Beispielsweise kommen das Hydrochlorid, Maleat, Sulfat und Gluconat von Flupirtin in Frage.

Die Gehalte an Flupirtin in den erfindungsgemäßen Arzneimitteln betragen 0,1 mg-3000 mg, vorzugsweise 10 mg-500 mg. Die genannten Einzeldosen des Arzneimittels können 1 - 5 mal, vorzugsweise 1 - 3 mal täglich verabreicht werden.

Die Dosierungsangaben beziehen sich immer auf Flupirtin als Base. Werden Salze des Flupirtins eingesetzt, so ist entsprechend dem Molgewicht umzurechnen.

Die pharmazeutische und galenische Handhabung der erfindungsgemäßen Verbindungen erfolgt nach den üblichen Standardmethoden. Beispielsweise wird Flupirtin und die Träger- und/oder Hilfsstoffe durch Rühren oder Homogenisieren gut vermischt, wobei im allgemeinen bei Temperaturen zwischen 20 und 80°C, vorzugsweise 20 bis 50°C, gearbeitet wird.

## Patentansprüche

1. Verwendung des Wirkstoffes Flupirtin oder dessen pharmazeutisch verwendbaren Salze zur Herstellung eines Arzneimittels zur Therapie und Prophylaxe von Myokardinfarkt.

2. Verwendung des Wirkstoffes Flupirtin oder dessen pharmazeutisch verwendbaren Salze zur Herstellung eines Arzneimittels zur Therapie und Prophylaxe von Schockniere.

3. Verwendung des Wirkstoffes Flupriting oder dessen pharmazeutisch verwendbaren Salze zur Herstellung eines Arzneimittels zur Therapie und Prophylaxe von Schocklunge.

## Claims

1. Use of the active compound flupirtine or its pharmaceutically utilizable salts for the production of a medicament for the therapy and prophylaxis of myocardial infarct.

2. Use of the active compound flupirtine or its pharmaceutically utilizable salts for the production of a medicament for the therapy and prophylaxis of shock kidney.

3. Use of the active compound flupirtine or its pharamceutically utilizable salts for the production of a medicament for the therapy and prophylaxis of shock lung.

## Revendications

1. Utilisation de la substance active Flupirtine ou de ses sels pharmaceutiquement utilisables pour la préparation d'un médicament pour la thérapie et la prophylaxie d'un infarctus du myocarde.

2. Utilisation de la substance active Flupirtine ou de ses sels pharmaceutiquement utilisables pour la préparation d'un médicament pour la thérapie et la prophylaxie de reins de choc.

3. Utilisation de la substance active Flupirtine ou de ses sels pharmaceutiquement utilisables pour la préparation d'un médicament pour la thérapie et la prophylaxie de poumons de choc.
